# EUROPÄISCHE PATENTSCHRIFT

(11) **EP 0 968 695 B1**
(45) Veröffentlichungstag und Bekanntmachung des Hinweises auf die Patenterteilung: **25.08.2004**
(21) Anmeldenummer: 99110089.2
(22) Anmeldetag: 22.05.1999
(51) Int. Cl.: A61F 13/08, A61F 2/50, A61F 2/78

(54) **Kosmetikstrumpf**
Cosmetic stocking
Bas cosmétique

(30) Priorität: 29.06.1998 DE 29811426 U
(43) Veröffentlichungstag der Anmeldung: 05.01.2000
(73) Patentinhaber: Otto Bock HealthCare GmbH, 37115 Duderstadt (DE)
(72) Erfinder: Stender, Adolf, 37115 Duderstadt (DE); Erlwein, Ludwig, 96146 Altendorf (DE); Koch, Thilo, 37115 Duderstadt (DE); Schlomski, Jens, 37115 Duderstadt (DE)
(74) Vertreter: Gramm, Werner, Prof. Dipl.-Ing.

(56) Entgegenhaltungen:
- FR-A- 1 304 470
- US-A- 5 133 775
- US-A- 5 529 830
- DIN 60900-Teil 2
- Hans J. Koslowski "Chemiefaserlexicon" S. 279, 280

## Beschreibung

Die Erfindung betrifft einen Kosmetikstrumpf zum Aufziehen auf eine Schaumstoffkosmetik einer Ober- und Unterschenkelprothese, mit einem das obere Strumpfende abschließenden Haftband zur Festlegung des Strumpfes am Prothesenschaft.

Zur möglichst weitgehenden Wiederherstellung des äußeren Erscheinungsbildes eines Prothesenträgers kommen für modular aufgebaute Prothesen mit Schaumstoffkosmetik handelsübliche Feinstrümpfe als Kosmetikstrümpfe zum Einsatz. Diese Feinstrümpfe haben den Nachteil, daß die Farbgebung von der Strumpffarbe abhängt, die Nachbildung der Haut nicht gelingt, und daß äußere Einflüsse durch den Strumpf hindurch in die Schaumkosmetik dringen und zusätzlich auch die Prothese selbst schädigen oder verändern können.

Bekannt ist ferner, die Schaumstoffkosmetik unmittelbar zu beschichten und so deren Erscheinungsbild zu verbessern. Bekanntgeworden sind weiterhin sogenannte Kosmetikhäute, die in umständlicher Weise durch Anwärmen auf die Schaumstoffkosmetik durch Anschrumpfen appliziert oder aber aufgeklebt werden müssen.

Aus der US-A-5,529,830 ist ein dehnbares Schichtstoff-Gewebe bekannt, das luftundurchlässig und wasserdicht, jedoch wasserdampfdurchlässig ist. Das Gewebe besteht aus einer Lage eines hydrophilen wasserdampfdurchlässigen synthetischen Polymers, das beidseitig mit einem dehnbaren Verbundwerkstoff beschichtet ist, der aus einer hydrophoben Lage eines porösen polymerischen Materials besteht. Aus diesem Gewebe kann z. B. Schutzkleidung hergestellt werden.

Der Erfindung liegt die Aufgabe zugrunde, den Kosmetikstrumpf hinsichtlich seiner Eigenschaften und seiner Herstellung zu verbessern.

Ausgehend von dem eingangs beschriebenen Kosmetikstrumpf wird diese Aufgabe erfindungsgemäß dadurch gelöst, daß der Strumpf als Kompressionsstrumpf ausgeführt ist, dessen Maschen im Knöchelbereich enger und im Oberschenkelbereich weiter gestrickt sind, und daß der Strumpf eine die Elastizität und Kompression des Grundgestricks erhaltende PUR-Beschichtung aufweist, die von innen nach außen dampfdurchlässig ist und sich zusammensetzt aus einer das Gestrick nicht durchdringenden, sondern auf dem Gestrick haftenden wasserdichten Grundierung und einer einen samtartigen Griff aufweisenden Deckschicht.

Ein erfindungsgemäßer Kosmetikstrumpf kann dadurch hergestellt werden, daß das Gestrick auf einer der Kontur des menschlichen Beins entsprechenden, demgegenüber aber etwas Untermaß aufweisenden Aufnahmedorn gezogen und dann über eine Spritzpistole beschichtet wird unter separater Material- und Preßluftzufuhr.

Verwendet wird somit ein formgestrickter Kompressionsstrumpf, dessen Form über die Stricktechnik, nicht aber durch z. B. Wärmebehandlung eingebracht wird. Die Verwendung eines Kompressionsstrumpfes führt zur Formung einer natürlichen Beinform, also zu einer engen Anlage im Knöchelbereich und einer weiten Form im Oberschenkelbereich. Die Übergänge zwischen dem z. B. aus Kunststoff bestehenden Prothesenfuß und der Schaumkosmetik werden egalisiert. Die hohe Elastizität des Gestricks erleichtert ein Anziehen des Strumpfes. Die Beschichtung ist erfindungsgemäß so gewählt, daß die Elastizität und Kompression des Grundgestricks erhalten bleibt. Dabei soll die Beschichtung zumindest spritzwasserfest eingestellt werden. Der beschichtete Strumpf darf die Kniebeugung des Kniegelenks nicht behindern und soll die Faltenbildung der Schaumkosmetik reduzieren. Da die Grundierung lediglich auf dem Gestrick haftet, es aber nicht bis auf seine Innenseite durchdringt, bleibt der Kosmetikstrumpf hautfreundlich.

Dabei ist es zweckmäßig, wenn das Gestrick eine Maschenreihe Polyamid, dtex 22f7x2 texturiert, und eine Maschenreihe Lycra, dtex 78 mit dtex 17f5 doppelt umwunden, aufweist. Handelsüblich spricht man hier von einer 70DEN-Kompression.

Die Grundierung hat die Funktion, eine optimale Haftung zum Strumpf sowie Wasserdichtigkeit zu erzielen. Die Verbindung Grundierung/Strumpf basiert nicht auf einer formschlüssigen Verbindung, sondern auf einer Haftung.

Dabei ist es zweckmäßig, wenn die Grundierung eine Schichtdicke von etwa 30 bis 40 µm aufweist, und wenn die Deckschicht eine Schichtdicke von etwa 50 bis 60 µm aufweist.

Bestimmte Problemzonen müssen speziell gefertigt und beschichtet werden. So ist es zweckmäßig, wenn das Haftband mit dem Strumpf über eine 3-Nadeldoppelkettenstichnaht verbunden ist, und wenn besonders belastete Zonen, wie z. B. der Fersen- und Zehenbereich, mit einer größeren Schichtstärke versehen werden.

Erfindungsgemäß weist die Grundierung vorzugsweise eine Schichtstärke von etwa 30 bis 40 µm und die Deckschicht eine Schichtstärke von 50 bis 60 µm auf, d. h. die gesamte Schichtdicke beträgt erfindungsgemäß ca. 80 bis 100 µm.

Das Gestrick besteht aus etwa 84 % Polyamid und etwa 16 % Elastan und weist eine Maschenreihe Polyamid, dtex 22f7x2 texturiert, und eine Maschenreihe Lycra, dtex 78 mit dtex 17f5 doppelt umwunden, auf.

Die Grundierung basiert auf einer Polyesterurethan-Zubereitung, während die Deckschicht ein Polyesterurethan-Addukt ist auf Basis von Toluylendiisocyanat, vernetzt durch Zusatz von Polyisocyanat.

Hinsichtlich der Herstellung ist es zweckmäßig, wenn der Material-Zuführdruck etwa 0,7 bis 1 bar und der Luftzerstäubungsdruck etwa 6 bar beträgt. Die Spritzpistole ist also mit einem Materialbehälter und einem Preßluftnetz verbunden und weist somit zwei Zuleitungen auf.

Durch einfaches Aufziehen des erfindungsgemäßen Kosmetikstrumpfes auf die vorhandene Schaumstoffkosmetik kann der versorgende Orthopädie-Techniker auf einen zusätzlichen Arbeitsgang, nämlich auf eine Beschichtung der Kosmetik verzichten. Außerdem kann der Patient selbst den beschichteten Strumpf aufziehen. Durch die vorhandene Umfangs- und Längsspannung ist der Strumpf in der Lage, gewisse Oberflächen- und Volumenänderungen zu überbrücken, ohne Falten zu bilden. Durch die nach handelsüblicher Größen-Tabelle voreingestellte Kompression wird eine sehr hohe Gleichmäßigkeit der Oberfläche, d. h. Faltenfreiheit erreicht. Gleichzeitig schützt die applizierte Schicht vor Umwelteinflüssen wie Feuchtigkeit, Schmutz, UV-Strahlung, Abrieb etc. Die auf aliphatischer Basis beruhende Beschichtung ist atmungsaktiv und dampfdurchlässig.

Die mittels der vorstehend beschriebenen druckluftbeaufschlagten Spritzpistole erzeugte Beschichtung kann manuell oder maschinell durchgeführt werden.

## Patentansprüche

1. Kosmetikstrumpf zum Aufziehen auf eine Schaumstoffkosmetik einer Ober- und Unterschenkelprothese, mit einem das obere Strumpfende abschließenden Haftband zur Festlegung des Strumpfes am Prothesenschaft, **dadurch gekennzeichnet, daß** der Strumpf als Kompressionsstrumpf ausgeführt ist, dessen Maschen im Knöchelbereich enger und im Oberschenkelbereich weiter gestrickt sind, und daß der Strumpf eine die Elastizität und Kompression des Grundgestricks erhaltende PUR-Beschichtung aufweist, die von innen nach außen dampfdurchlässig ist und sich zusammensetzt aus einer das Gestrick nicht durchdringenden, sondern auf dem Gestrick haftenden wasserdichten Grundierung und einer einen samtartigen Griff aufweisenden Deckschicht.

2. Kosmetikstrumpf nach Anspruch 1, **dadurch gekennzeichnet, daß** die Grundierung eine Schichtdicke von etwa 30 bis 40 µm aufweist.

3. Kosmetikstrumpf nach Anspruch 1 oder 2, **dadurch gekennzeichnet, daß** die Deckschicht eine Schichtdicke von etwa 50 bis 60 µm aufweist.

4. Kosmetikstrumpf nach Anspruch 3, **dadurch gekennzeichnet, daß** der Fersen- und Zehenbereich eine größere Schichtstärke aufweist.

5. Kosmetikstrumpf nach einem der vorhergehenden Ansprüche, **dadurch gekennzeichnet, daß** das Gestrick aus etwa 84 % Polyamid und etwa 16 % Elastan besteht.

6. Kosmetikstrumpf nach einem der vorhergehenden Ansprüche, **dadurch gekennzeichnet, daß** das Gestrick eine Maschenreihe Polyamid, dtex 22f7x2 texturiert, und eine Maschenreihe Lycra, dtex 78 mit dtex 17f5 doppelt umwunden, aufweist.

7. Kosmetikstrumpf nach einem der vorhergehenden Ansprüche, **dadurch gekennzeichnet, daß** die Grundierung auf einer Polyesterurethan-Zubereitung basiert.

8. Kosmetikstrumpf nach einem der vorhergehenden Ansprüche, **dadurch gekennzeichnet, daß** die Deckschicht ein Polyesterurethan-Addukt ist auf Basis von Toluylendiisocyanat,vernetzt durch Zusatz von Polyisocyanat.

9. Kosmetikstrumpf nach einem der vorhergehenden Ansprüche, **dadurch gekennzeichnet, daß** das Haftband mit dem Strumpf über eine 3-Nadeldoppelkettenstichnaht verbunden ist.

## Claims

1. Cosmetic stocking for pulling onto a cosmetic foam of an above-knee and below-knee prosthesis, having a gripper band which terminates the upper end of the stocking and is adapted for securing the stocking to the shaft of the prosthesis, **characterized in that** the stocking is constructed as a compression stocking whose stitches are loop-drawingly knitted to be narrower in the ankle region and wider in the above-knee region, and **in that** the stocking comprises a polyurethane coating which preserves the elasticity and compression of the ground knit and which is vapour pervious from in to out and is composed of a water-impervious primer coat which does not penetrate through the knit, but adheres to the knit, and a top coat which has a velvety handle.

2. Cosmetic stocking according to Claim 1, **characterized in that** the primer coat is about 30 to 40 *µ*m in layer thickness.

3. Cosmetic stocking according to Claim 1 or 2, **characterized in that** the top coat is about 50 to 60 *µ*m in layer thickness.

4. Cosmetic stocking according to Claim 3, **characterized in that** the heel and toe region has a higher layer thickness.

5. Cosmetic stocking according to any one of the preceding claims, **characterized in that** the knit is about 84% polyamide and about 16% elastane.

6. Cosmetic stocking according to any one of the preceding claims, **characterized in that** the knit comprises a course of textured twofold 22 dtex 7 filament polyamide and a course of 78 dtex Lycra double covered with 17 dtex 5 filament.

7. Cosmetic stocking according to any one of the preceding claims, **characterized in that** the primer coat is based on a polyester urethane composition.

8. Cosmetic stocking according to any one of the preceding claims, **characterized in that** the top coat is a polyester urethane adduct based on tolylene diisocyanate and crosslinked by addition of polyisocyanate.

9. Cosmetic stocking according to any one of the preceding claims, **characterized in that** the gripper band is joined to the stocking via a 3 needle double chain stitch seam.

## Revendications

1. Bas cosmétique destiné à être enfilé sur une cosmétique en mousse d'une prothèse de la jambe supérieure et inférieure, le bas comportant une bande adhérante terminant l'extrémité supérieure du bas pour fixer le bas au fût de la prothèse, **caractérisé en ce que** le bas est réalisé sous la forme d'un bas de compression, dont les mailles sont tricotées plus étroitement dans la région de la cheville et plus largement dans la région de la jambe supérieure, et **en ce que** le bas comporte un revêtement PUR conservant l'élasticité et la compression du tricot de base, ce revêtement étant perméable à la vapeur de l'intérieur vers l'extérieur et se composant d'un apprêt étanche à l'eau ne pénétrant pas le tricot mais adhérant sur le tricot, et d'une couche de couverture présentant un toucher du type velours.

2. Bas cosmétique selon la revendication 1, **caractérisé en ce que** l'apprêt présente une épaisseur de couche d'environ 30 à 40 µm.

3. Bas cosmétique selon la revendication 1 ou 2, **caractérisé en ce que** la couche de couverture présente une épaisseur de couche d'environ 50 à 60 µm.

4. Bas cosmétique selon la revendication 3, **caractérisé en ce que** la région du talon et de la pointe présente une plus grande épaisseur de couche.

5. Bas cosmétique selon l'une des revendications précédentes, **caractérisé en ce que** le tricot est constitué d'environ 84% de polyamide et environ 16% d'élastane.

6. Bas cosmétique selon l'une des revendications précédentes, **caractérisé en ce que** le tricot présente une rangée de mailles de polyamide, texturée dtex 22f7x2, et une rangée de mailles de lycra, dtex 78 doublement enveloppé de dtex 17f5.

7. Bas cosmétique selon l'une des revendications précédentes, **caractérisé en ce que** l'apprêt est à base d'une préparation polyesteruréthane.

8. Bas cosmétique selon l'une des revendications précédentes, **caractérisé en ce que** la couche de couverture est un produit d'addition polyesteruréthane à base de diisocyanate de toluylène, réticulé par apport de polyisocyanate.

9. Bas cosmétique selon l'une des revendications précédentes, **caractérisé en ce que** la bande adhérante est reliée au bas par une couture au double point de chaînette à trois aiguilles.
